# EUROPEAN PATENT SPECIFICATION

(11) **EP 2 962 719 B1**
(45) Date of publication and mention of the grant of the patent: **18.12.2019**
(21) Application number: 15170290.9
(22) Date of filing: 02.06.2015
(51) Int. Cl.: A61M 25/10

(54) **BALLOON CATHETER**
BALLONKATHETER
CATHÉTER À BALLONNET

(30) Priority: 01.07.2014 JP 2014135567
(43) Date of publication of application: 06.01.2016
(73) Proprietor: Asahi Intecc Co., Ltd., Seto-shi, Aichi 489-0071 (JP)
(72) Inventor: Nakagawa, Yuta, Seto-shi, Aichi 489-0071 (JP); Kubo, Yuta, Seto-shi, Aichi 489-0071 (JP)
(74) Representative: Winter, Brandl, Fürniss, Hübner, Röss, Kaiser, Polte - Partnerschaft mbB

(56) References cited:
- WO-A1-2009/146285
- US-A- 5 853 389
- US-A1- 2005 261 723
- US-A1- 2007 129 748
- US-A1- 2013 303 983

## Description

### TECHNICAL FIELD

The present invention relates to a balloon catheter for securing the flow of blood, bile (gall), pancreatic fluid and the like by inserting it into a stenosis site or a obstructed segment formed in a blood vessel, bile duct, pancreatic duct and the like to expand the stenosis site or the obstructed segment.

### BACKGROUND ART

When a stenosis site or an obstructed segment is formed in a blood vessel, bile duct, pancreatic duct and the like, the flow of blood, bile (gall), pancreatic fluid and the like becomes hindered. Traditionally, treatments with a balloon catheter have been widely performed to treat such a stenosis site or an obstructed segment.

A balloon catheter mainly comprises a balloon as an expandable body, an outer tube fixed to the proximal end of the balloon and an inner tube inserted into the balloon and the outer tube. The inner tube is used for inserting a guide wire through it. An inflation lumen provided between the outer tube and the inner tube is used to pass a liquid (a contrast medium, physiological saline and the like) to expand the balloon.

Usually, a balloon catheter is inserted into a blood vessel, bile duct, pancreatic duct and the like with a balloon folded around the outer periphery of an inner tube, and the balloon is expanded at a stenosis site or an obstructed segment. Then the balloon is again folded around the outer periphery of the inner tube. Therefore, the balloon transforms a transition from a folded state to an expanded state, or from an expanded state to a folded state. In a case where there are multiple stenosis site or obstructed segments, and the length of a stenosis site or an obstructed segment is longer than that of a balloon, an operator repeats the above operations. As a result, if balloon shaping is not correctly performed, the diameter of the balloon may not become small enough when folded, resulting in a problem that the operativity of the balloon catheter gradually decreases.

As a method of solving this problem, known is a balloon catheter in which a balloon is pre-shaped so that the balloon regularly has n (n is an integer of 2 or more) wing portions and n (n is an integer of 2 or more) valley portions formed between adjacent wing portions when folded around the outer periphery of an inner tube (for example, see Japanese Patent No. 4761671; WO2009/146285 A1; US5853389 A; US2013/303983 A1).

US 2007/129748 A1 and US 2005/261723 A1 disclose balloons with "bat wings" or "accordion wings" where each wing is folded so that a plurality of valley portions are formed in a radial direction.

However, in the case of the balloon catheter according to Japanese Patent No. 4761671, distances between the n valley portions and the inner tube are all the same since the balloon is pre-shaped so that it has n wing portions and n valley portions in a regular fashion when folded. Therefore, a certain pressure (pressurization or depressurization) is only exerted evenly on each of the n valley portions when the balloon is allowed to transform from a folded state to an expanded state, or when the balloon is allowed to transform from an expanded state to a folded state. In particular, when a balloon catheter is inserted up to the end of a curved blood vessel, bile duct, pancreatic duct and the like, considerable time is required until a pressure (depressurization) to fold a valley portion of a balloon, or a pressure (pressurization) to expand a valley portion of the balloon is exerted on each of the n valley portions. Therefore, a time required to allow a balloon to transform from a folded state to an expanded state becomes longer, or a time required to allow a balloon to transform from an expanded state to a folded state becomes longer. As a result, disadvantageously, a long time is required to expand a stenosis site or an obstructed segment.

### SUMMARY OF THE INVENTION

### TECHNICAL PROBLEM

The present invention is made in view of the circumstances described above. An object of the present invention is to provide a balloon catheter having a balloon capable of transforming from an expanded state to a folded state or from a folded state to an expanded state in a short time.

### SOLUTION TO PROBLEM

The above problems can be solved by the means listed below.

An aspect 1 of the present invention is a balloon catheter comprising an inner tube and a balloon fixed to the inner tube, the balloon being capable of transforming a transition between a folded state and an expanded state, wherein the balloon has n (n is an integer of 2 or more) wing portions and n (n is an integer of 2 or more) valley portions formed between adjacent wing portions when the balloon is folded around the outer periphery of the inner tube, the n wing portions and the n valley portions are arranged in a circumferential direction and a distance between at least one valley portion of the n valley portions and the inner tube is shorter than distances between other valley portions and the inner tube.

An aspect 2 of the present invention is the balloon catheter according to aspect 1, having a portion where a distance between an n-th valley portion (n is an integer of 3 or more) and the inner tube is shorter than a distance between an (n-1)th valley portion adjacent to the n-th valley portion and the inner tube, and a distance between an (n-2)th valley portion adjacent to the (n-1)th valley portion and the inner tube is shorter than a distance between the (n-1)th valley portion and the inner tube.

An aspect 3 of the present invention is the balloon catheter according to aspect 1 or 2, wherein the n valley portions comprise a short valley portion having a short distance to the inner tube and a long valley portion having a long distance to the inner tube, and the short valley portion and the long valley portion are alternately arranged in a circumferential direction.

### ADVANTAGEOUS EFFECT OF THE INVENTION

In the balloon catheter according to the aspect 1 of the present invention, a distance between at least one valley portion of the n valley portions and the inner tube is shorter than distances between other valley portions to the inner tube. Therefore, when a balloon is allowed to transform from an expanded state to a folded state, a valley portion having a short distance to the inner tube is depressurized more preferentially than a valley portion having a long distance to the inner tube, and starts to fold first. This also triggers folding of the other valley portions having long distances to the inner tube by being dragged. Therefore, the other valley portions having long distances to the inner tube start to fold before a pressure (depressurization) for folding is exerted. This can shorten a time required to allow the balloon to transform from an expanded state to a folded state. Similarly, when the balloon is allowed to transform from a folded state to an expanded state, the other valley portions having long distances to the inner tube are pressurized more preferentially than the valley portion having a short distance to the inner tube and start to expand first. This also triggers expansion of the valley portion having a short distance to the inner tube by being dragged. Therefore, the valley portion having short distance to the inner tube starts to expand before a pressure (pressurization) for expansion is exerted. This can shorten a time required to allow the balloon to transform from a folded state to an expanded state.

The balloon catheter according to the aspect 2 of the present invention has a portion where the distance between an n-th valley portion and the inner tube is shorter than that between an (n-1)th valley portion adjacent to the n-th valley portion and the inner tube, and the distance between an (n-2)th valley portion adjacent to the (n-1)th valley portion and the inner tube is shorter than that between the (n-1)th valley portion and the inner tube. Therefore, when a balloon is allowed to transform from an expanded state to a folded state, the n-th valley portion and the (n-2)th valley portion having a short distance to the inner tube is depressurized more preferentially than the (n-1)th valley portion having a long distance to the inner tube, and starts to fold first. The (n-1)th valley portion starts to fold by being dragged from both sides since it is sandwiched between the n-th valley portion and the (n-2)th valley portion. As a result, a time required to allow the balloon to transform from an expanded state to a folded state can be further shortened.

In the balloon catheter according to the aspect 3 of the present invention, the n valley portions comprise a short valley portion having a short distance to the inner tube and a long valley portion having a long distance to the inner tube, and the short valley portion and the long valley portion are alternately arranged in a circumferential direction. Therefore, when a balloon is allowed to transform from an expanded state to a folded state, the valley portion having a short distance to the inner tube starts to preferentially fold evenly in the circumferential direction. The valley portion having a long distance to the inner tube starts to fold evenly in the circumferential direction by being dragged from both sides since it is sandwiched between the valley portions having a short distance to the inner tube. As a result, the balloon can fold without becoming eccentric relative to the inner tube, and a time required to allow the balloon to transform from an expanded state to a folded state can be further shortened. Similarly, when the balloon is allowed to transform from a folded state to an expanded state, the valley portion having a long distance to the inner tube starts to preferentially expand evenly in the circumferential direction. The valley portion having a short distance to the inner tube starts to expand evenly in the circumferential direction by being dragged from both sides since it is sandwiched between the valley portions having a long distance to the inner tube. As a result, the balloon can expand without becoming eccentric to the inner tube, and a time required to allow the balloon to transform from a folded state to an expanded state can be further shortened.

### BRIEF DESCRIPTION OF THE DRAWINGS

Fig. 1 shows an overall view of a balloon catheter (with a balloon expanded) according to an embodiment of the present invention.
Fig. 2 shows the A-A cross section in Fig. 1.
Fig. 3 shows an overall view of the balloon catheter (with the balloon folded) according to the embodiment of the present invention.
Fig. 4 shows the B-B cross section in Fig. 3.
Fig. 5 shows how the balloon transforms a transition from an expanded state to a folded state. Fig. 5A shows a state where the balloon is expanded. Fig. 5B shows a state where a valley portion having a short distance to the inner tube starts to preferentially fold. Fig. 5C shows a state where valley portions having a long distance to the inner tube start to fold by being dragged. Fig. 5D shows a state where the 6 valley portions are all folded.
Fig. 6 shows a first variation of Fig. 4.
Figs. 7A to 7D show a first variation of Figs. 5A to 5D.
Fig. 8 shows a second variation of Fig. 4.
Figs. 9A to 9D show a second variation of Figs. 5A to 5D.

### DESCRIPTION OF EMBODIMENTS

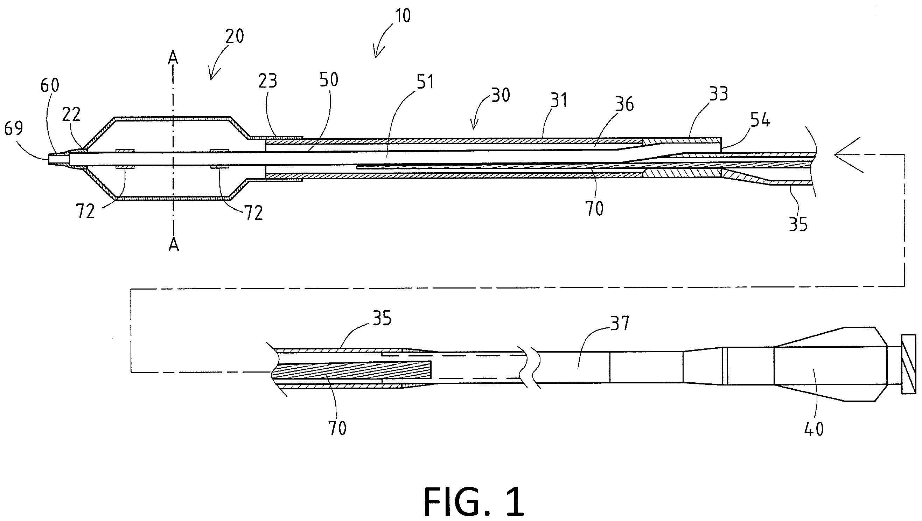

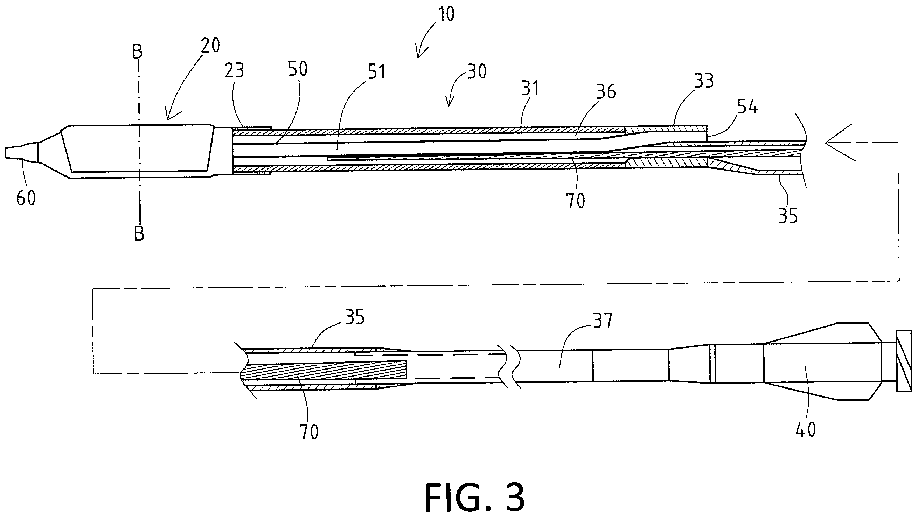

The present invention will be described with reference to Figs. 1 to 5D using a case where a balloon catheter 10 according to an embodiment of the present invention is an example. The left side in Figs. 1 and 3 corresponds to the distal end side (the front end side) which is to be inserted into the body, and the right side corresponds to the proximal end side (the base end side) which is to be operated by an operator such as a physician.

The balloon catheter 10 may be used for treating, for example, a stenosis site or an obstructed segment formed in a blood vessel, bile duct, pancreatic duct and the like. As shown in Fig. 1, the balloon catheter 10 mainly comprises a balloon 20, an outer tube 30, a connector 40, an inner tube 50, a tip 60 and a reinforcer 70. Note that Fig. 1 shows the balloon 20 in an expanded state.

The balloon 20 for expanding a stenosis site or obstructed segment comprises a resin material, and has a distal attaching portion 22 at the distal end side and a proximal attaching portion 23 at the proximal end side. The distal attaching portion 22 is fixed to the distal end of the inner tube 50 and the tip 60, and the proximal attaching portion 23 is fixed to the distal end of the outer tube 30. Although the distal attaching portion 22 is fixed to the distal end of the inner tube 50 through the tip 60 in Fig. 1, the configuration is not limited to this. The distal attaching portion 22 may be sandwiched between the distal end of the inner tube 50 and the tip 60. Further, although the proximal attaching portion 23 is fixed to the outer periphery of the outer tube 30 at the distal end in Fig. 1, the configuration is not limited to this. The proximal attaching portion 23 may be fixed to the inner periphery of the outer tube 30 at the distal end.

The outer tube 30 is a tubular member constituting an inflation lumen 36 for supplying a liquid such as a contrast medium or a physiological saline in order to expand the balloon 20. The outer tube 30 comprises a distal end outer tube part 31, a guide wire port part 33, a middle outer tube part 35 and a proximal end outer tube part 37 in the order from the distal end side. The distal end outer tube part 31 and the middle outer tube part 35 are tubes comprising a resin such as polyamide, polyamide elastomer, polyolefine, polyester and polyester elastomer. The guide wire port part 33 is a portion to which the distal end outer tube part 31, the middle outer tube part 35 and the inner tube 50 are fixed.

The inner tube 50 is inserted into the distal end outer tube part 31, and the aforementioned inflation lumen 36 is formed between the distal end outer tube part 31 and the inner tube 50.

The proximal end outer tube part 37 is a metal tubular member which is so-called a "hypo tube." The distal end of the proximal end outer tube part 37 is inserted into and fixed to the proximal end of the middle outer tube part 35. The connector 40 is attached to the proximal end of the proximal end outer tube part 37. When a liquid for expanding the balloon 20 such as a contrast medium or a physiological saline is supplied from an indeflator (not shown) attachable to the connector 40, the liquid flows through the inflation lumen 36 to expand the balloon 20. Note that there is no particular limitation for the material of the proximal end outer tube part 37, and stainless steel (SUS304) and a superelastic alloy such as an Ni-Ti alloy can be used.

The inner tube 50 forms a guide wire lumen 51 for inserting a guide wire inside. Further, the proximal end of the inner tube 50 forms a proximal side guide wire port 54 by being fixed to the guide wire port part 33 of the outer tube 30.

The distal end of the inner tube 50 is fixed to the tip 60 and the distal attaching portion 22 of the balloon 20. The tip 60 is a member having a tapered external shape in which the outer diameter gradually decreases toward the distal end, and is formed with a flexible resin. There is no particular limitation for the resin for forming the tip 60, and polyurethane, polyurethane elastomer and the like can be used.

The tip 60 is a cylindrical member fixed to the distal end of the guide wire lumen 51, and has the distal guide wire port 69 at the distal end.

Two radiopaque markers 72 are attached to the inner tube 50 inside the balloon 20 so that the position of the balloon 20 can be detected under radiation.

The reinforcer 70 is attached to the inner periphery of the proximal end outer tube part 37 at the distal end. The reinforcer 70 has a circular cross section, and is a tapered metal wire member in which the diameter decreases toward the distal end. There is no particular limitation for the material of the reinforcer 70, and stainless steel (SUS304) and a superelastic alloy such as an Ni-Ti alloy can be used.

The reinforcer 70 passes through the middle outer tube part 35 and the guide wire port part 33, and extends to the distal end outer tube part 31. The distal end of the reinforcer 70 is fixed to the outer tube 30 and the inner tube 50 in Fig. 1, but the configuration is not limited to this. For example, the distal end of the reinforcer 70 may be fixed so that it is sandwiched between the outer tube 30 and the inner tube 50.

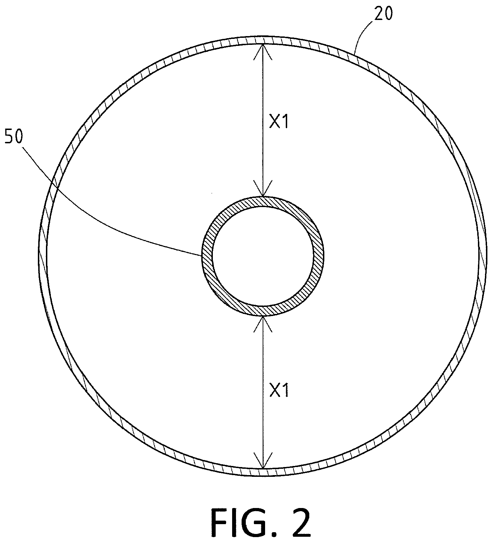

Fig. 2 shows the A-A cross section in Fig. 1. As shown in Fig. 2, when a liquid such as a contrast medium or a physiological saline is supplied to the balloon 20, the balloon 20 is expanded centrally around the inner tube 50 over the circumference. At this time, the distance between the balloon 20 and the inner tube 50 is defined as X1.

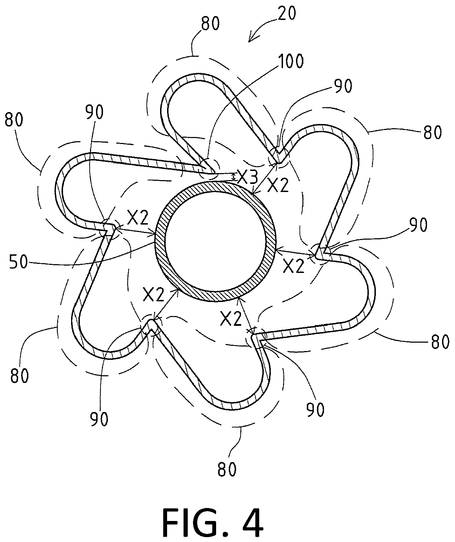

Fig. 3 shows a state in which the balloon 20 is folded around the outer periphery of the inner tube 50. Note that unlike Fig. 1, a portion from the tip 60 to the balloon 20 is shown as an external view in Fig. 3 instead of a cross sectional view for the purposes of illustration. Fig. 4 shows the B-B cross section in Fig. 3. As shown in Fig. 4, the balloon 20 has 6 wing portions 80, and 6 valley portions 90, 100 formed between adjacent wing portions 80 when the balloon 20 is folded around the outer periphery of the inner tube 50. The 6 valley portions 90, 100 are grouped into two groups: 5 valley portions 90 having a long distance X2 to the inner tube 50 and one valley portion 100 having a short distance X3 to the inner tube 50 (X2 > X3).

In this embodiment of the present invention, 6 valley portions 90 having a long distance X2 to the inner tube 50 were loosely shaped by loosely pressurizing the balloon 20 in a mold. Subsequently, a valley portion 100 having a short distance X3 to the inner tube 50 was produced by wrapping one valley portion 90 of the 6 valley portions 90 and performing physically firm shaping. However, there is no particular limitation for the method of differentially producing the valley portions 90 and the valley portion 100. For example, a loosely shaped valley portion 90 having a long distance X2 to the inner tube 50 and a firmly shaped valley portion 100 having a short distance X3 to the inner tube 50 may be produced by pressurizing the balloon 20 in a mold already having the valley portion 90 having the long distance X2 to the inner tube 50 and the valley portion 100 having the short distance X3 to the inner tube 50.

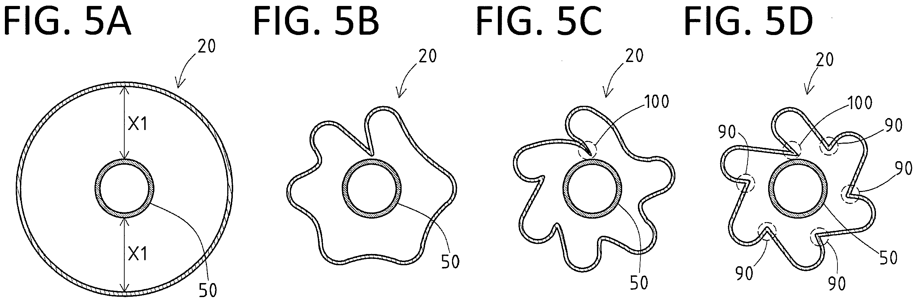

Figs. 5A to 5D show how the balloon 20 transforms a transition from an expanded state to a folded state. Note that it is described with reference to the valley portions 90, 100 of the balloon 20 in Figs. 5A to 5D for clear understanding. Therefore, description about the wing portion 80 is omitted. When a liquid such as a contrast medium or a physiological saline supplied to the balloon 20 is removed with an indeflator, a certain pressure (depressurization) is exerted on the balloon 20. At this time, the firmly shaped valley portion 100 is depressurized more preferentially than the loosely shaped valley portions 90, and starts to fold first (see Fig. 5B). The loosely shaped valley portions 90 then starts to fold by being dragged by the valley portion 100 when the valley portion 100 starts to fold (see Fig. 5C).

As described above, the balloon 20 has the loosely pre-shaped valley portions 90 having long distance X2 to the inner tube 50 and the firmly pre-shaped valley portion 100 having the short distance X3 to the inner tube 50 (see Fig. 4). As a result, the valley portion 100 preferentially folds and tries to drag the valley portions 90 when a certain pressure (depressurization) is exerted on the balloon 20. Therefore, a time required for allowing the balloon 20 to transform from an expanded state to a folded state can be shortened since the valley portion 90 starts to fold before a pressure (depressurization) for folding is applied.

Further, in a case where the balloon 20 is allowed to transform from a folded state to an expanded state (in other words, a transition from a state shown in Fig. 5D to a state shown in Fig. 5A), a certain pressure (pressurization) is exerted on the balloon 20 when a liquid such as a contrast medium or a physiological saline is supplied to the balloon 20 with an indeflator. At this time, the loosely shaped valley portions 90 are pressurized more preferentially than the firmly shaped valley portion 100, and start to expand first (see Fig. 5C). When the valley portions 90 start to expand, the firmly shaped valley portion 100 starts to expand by being dragged by the valley portions 90 (see Fig. 5B).

As described above, the balloon 20 has the loosely pre-shaped valley portions 90 having the long distance X2 to the inner tube 50 and the firmly pre-shaped valley portion 100 having the short distance X3 to the inner tube 50 (see Fig. 4). As a result, the valley portions 90 preferentially expand to drag the valley portion 100 when a certain pressure (pressurization) is exerted on the balloon 20. Therefore, a time required for allowing the balloon 20 to transform from a folded state to an expanded state can be shortened since the valley portion 100 starts to expand before a pressure (pressurization) for expanding is applied.

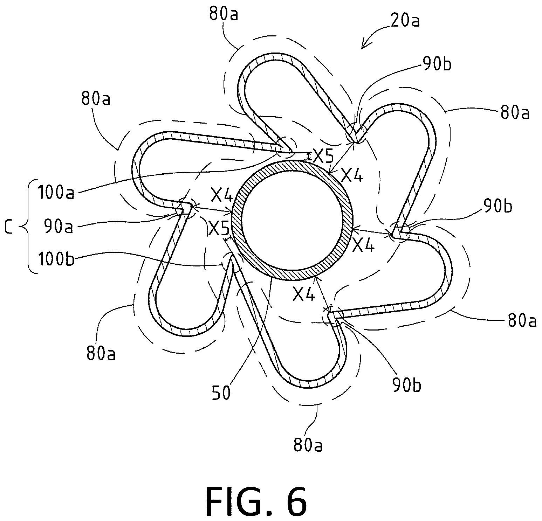

Fig. 6 shows a first variation of Fig. 4. When a balloon 20a is folded around the outer periphery of an inner tube 50, the balloon 20a has 6 wing portions 80a and 6 valley portions 90a, 90b, 100a, 100b formed between adjacent wing portions 80a. The 6 valley portions 90a, 90b, 100a, 100b are grouped into two groups: 4 valley portions 90a, 90b having a long distance X4 to the inner tube 50 and 2 valley portions 100a, 100b having a short distance X5 to the inner tube 50 (X4 > X5).

The balloon 20 shown in Fig. 4 has only one valley portion 100 having the short distance X3 to the inner tube 50. In contrast, the balloon 20a shown in Fig. 6 has 2 valley portions 100a, 100b having the short distance X5 to the inner tube 50 and the valley portion 90a having the long distance X4 to the inner tube 50 is provided between the 2 valley portions 100a, 100b. Therefore, when the balloon 20a is folded around the outer periphery of the inner tube 50, the balloon 20a has a portion where the distance X5 from the valley portion 100a to the inner tube 50 is shorter that the distance X4 from the adjacent valley portion 90b to the inner tube 50, and the distance X5 from the valley portion 100b adjacent the valley portion 90a to the inner tube 50 is shorter than the distance X4 from the valley portion 90a to the inner tube 50 (see Part C in Fig. 6).

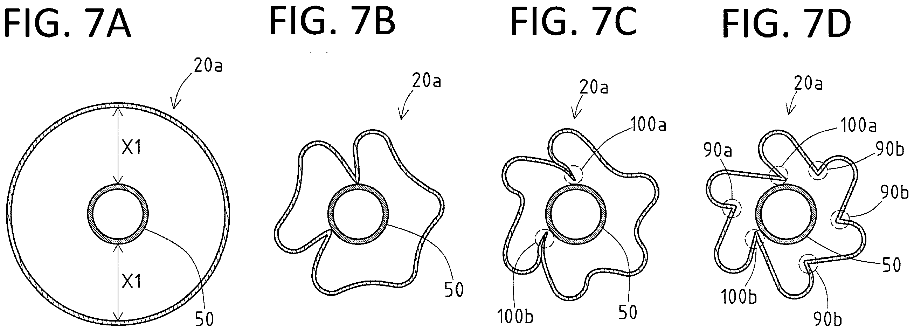

Figs. 7A to 7D show how the balloon 20a transforms a transition from an expanded state to a folded state. Note that it is described with reference to the valley portions 90a, 90b, 100a, 100b of the balloon 20a in Figs. 7A to 7D for clear understanding. Therefore, description about the wing portion 80a is omitted. When a liquid such as a contrast medium or a physiological saline supplied to the balloon 20a is removed with an indeflator, a certain pressure (depressurization) is applied to the balloon 20a. At this time, the firmly shaped valley portions 100a and 100b are depressurized more preferentially than the loosely shaped valley portion 90a, and start to fold first (see Fig. 7B). The valley portion 90a starts to fold by being dragged from both sides when the valley portions 100a and 100b at both sides start to fold since the valley portion 90a is sandwiched between the valley portions 100a and 100b (see Fig. 7C).

Therefore, the valley portions 100a and 100b preferentially fold and try to drag the valley portion 90a from both sides when a certain pressure (depressurization) is applied to the balloon 20a. Therefore, a time required for allowing the balloon 20a to transform from an expanded state to a folded state can be shortened since the valley portion 90a starts to fold before a pressure (depressurization) for folding is applied.

Further, in a case where the balloon 20a is allowed to transform from a folded state to an expanded state (in other words, a transition from a state shown in Fig. 7D to a state shown in Fig. 7A), a certain pressure (pressurization) is applied to the balloon 20a when a liquid such as a contrast medium or a physiological saline is supplied to the balloon 20a with an indeflator. At this time, the loosely shaped valley portion 90a is pressurized more preferentially than the firmly shaped valley portions 100a and 100b, and starts to expand first (see Fig. 7C). When the valley portion 90a starts to expand, the valley portions 100a and 100b at both sides start to expand by being dragged (see Fig. 7B).

In this way, the valley portion 90a preferentially expands and tries to drag the valley portions 100a and 100b when a certain pressure (pressurization) is applied to the balloon 20a. Therefore, a time required for allowing the balloon 20a to transform from a folded state to an expanded state can be further shortened since the valley portions 100a and 100b start to expand before a pressure (pressurization) for expanding is applied.

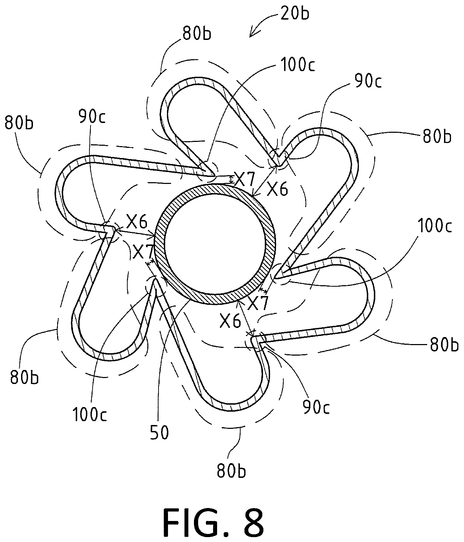

Fig. 8 shows a second variation of Fig. 4. in which the balloon 20b has 6 wing portions 80b and 6 valley portions 90c, 100c formed between adjacent wing portions 80b when a balloon 20b is folded around the outer periphery of an inner tube 50. The 6 valley portions 90c, 100c are grouped into two groups: 3 long valley portions 90c having a long distance X6 to the inner tube 50 and 3 short valley portions 100c having a short distance X7 to the inner tube 50 (X6 > X7), and the 3 long valley portions 90c and the 3 short valley portions 100c are alternately arranged in the circumferential direction.

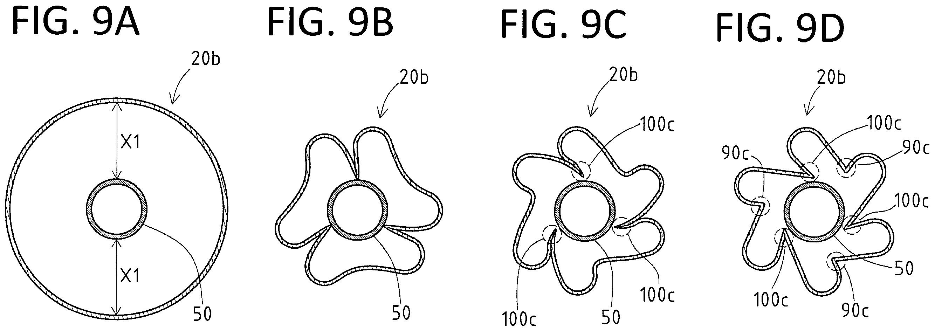

Figs. 9A to 9D show how the balloon 20b transforms a transition from an expanded state to a folded state. Note that it is described with reference to the valley portions 90c, 100c of the balloon 20b in Figs. 9A to 9D for clear understanding. Therefore, description about the wing portion 80b is omitted. When a liquid such as a contrast medium or a physiological saline supplied to the balloon 20b is removed with an indeflator, a certain pressure (depressurization) is applied to the balloon 20b. At this time, the firmly shaped short valley portions 100c are depressurized more preferentially than the loosely shaped long valley portions 90c, and start to fold evenly in a circumferential direction (see Fig. 9B). The loosely shaped long valley portions 90c start to fold evenly in the circumferential direction by being dragged from both sides to the short valley portions 100c when the short valley portions 100c start to fold (see Fig. 9C).

Therefore, the short valley portions 100c preferentially and evenly fold and try drag the long valley portions 90c sandwiched between the short valley portions 100c from both sides when a certain pressure (depressurization) is applied to the balloon 20b. As a result, the balloon 20b can fold without becoming eccentric relative to the inner tube 50, and a time required for allowing the balloon 20b to transform from an expanded state to a folded state can be shortened since the long valley portions 90c start to fold evenly before a pressure (depressurization) for folding is applied.

Further, in a case where the balloon 20b is allowed to transform from a folded state to an expanded state (in other words, a transition from a state shown in Fig. 9D to a state shown in Fig. 9A), a certain pressure (pressurization) is applied to the balloon 20b when a liquid such as a contrast medium or a physiological saline is supplied to the balloon 20b with an indeflator. At this time, the loosely shaped long valley portions 90c are pressurized more preferentially than the firmly shaped short valley portions 100c, and start to expand evenly in a circumferential direction (see Fig. 9C). When the short valley portions 90c start to expand, the firmly shaped short valley portions 100c start to expand by being dragged from both sides to the long valley portions 90c (see Fig. 9B).

As described above, the long valley portions 90c preferentially and evenly expand and try to drag the short valley portions 100c sandwiched between the long valley portions 90c from both sides when a certain pressure (pressurization) is applied to the balloon 20b. As a result, the balloon 20b can expand without becoming eccentric relative to the inner tube 50, and a time required for allowing the balloon 20b to transform from a folded state to an expanded state can be further shortened since the short valley portions 100c start to expand evenly before a pressure (pressurization) for expanding is applied.

Note that the balloons 20, 20a, 20b have 6 wing portions 80, 80a, 80b and 6 valley portions 90, 90a, 90b, 90c, 100, 100a, 100b, 100c as described above when the balloons 20, 20a, 20b are folded around the outer periphery of the inner tube 50 (see Figs. 4, 6 and 8). However, there is no particular limitation for the number of the wing portions 80, 80a, 80b and the valley portions 90, 90a, 90b, 90c, 100, 100a, 100b, 100c.

For example, the balloon 20 may have n (n is an integer of 2 or more) wing portions 80 and n (n is an integer of 2 or more) valley portions 90, 100 formed between adjacent wing portions 80 when the balloon 20 is folded around the outer periphery of the inner tube 50, and the distance X3 from at least one valley portion 100 of the n valley portions 90, 100 to the inner tube 50 may be shorter than the distances X2 from other valley portions 90 to the inner tube 50. Further, the balloon 20a may have a portion where the distance X5 between the n-th valley portion 100a and the inner tube 50 is shorter than the distance X4 between the adjacent (n-1)th valley portion 90a and the inner tube 50 when the balloon 20a is folded around the outer periphery of the inner tube 50, and the distance X5 between the (n-2)th valley portion 100b adjacent to the (n-1)th valley portion 90a and the inner tube 50 is shorter than the distance X4 between the (n-1)th valley portion 90a and the inner tube 50. Further, the short valley portions 100c having the short distance X7 to the inner tube 50 and the long valley portions 90c having the long distance X6 to the inner tube 50 may be alternately provided in the balloon 20b in a circumferential direction when the balloon 20b is folded around the outer periphery of the inner tube 50.

As described above, the distance between at least one valley portion 100, 100a, 100b, 100c of the n valley portions 90, 90a, 90b, 90c, 100, 100a, 100b, 100c and the inner tube 50 is shorter than distances between other valley portions 90, 90a, 90b, 90c and the inner tube 50 when the balloons 20, 20a, 20b of the balloon catheter 10 are folded around the outer periphery of the inner tube 50. As a result, the valley portions 100, 100a, 100b, 100c having a short distance to the inner tube 50 can be preferentially depressurized to quickly fold when the balloons 20, 20a, 20b are allowed to transform from an expanded state to a folded state, while the valley portions 90, 90a, 90b, 90c having a long distance to the inner tube 50 can be preferentially pressurized to quickly expand when the balloons 20, 20a, 20b are allowed to transform from a folded state to an expanded state.

### DESCRIPTION OF SYMBOLS

- 10: Balloon Catheter
- 20, 20a, 20b: Balloon
- 22: Distal attaching portion
- 23: Proximal attaching portion
- 30: Outer tube
- 31: Distal end outer tube part
- 33: Guide wire port part
- 35: Middle outer tube part
- 36: Inflation lumen
- 37: Proximal end outer tube part
- 40: Connector
- 50: Inner tube
- 51: Guide wire lumen
- 54: Proximal side guide wire port
- 60: Tip
- 69: Distal side guide wire port
- 70: Reinforcer
- 72: Marker
- 80, 80a, 80b: Wing portion
- 90, 90a, 90b, 90c: Valley portion with long distance
- 100, 100a, 100b, 100c: Valley portion with short distance

## Claims

1. A balloon catheter (10) comprising:
an inner tube (50); and
a balloon (20, 20a, 20b) fixed to the inner tube (50), the balloon (20, 20a, 20b) being capable of transforming a transition between a folded state and an expanded state,
**characterized in that**:
the balloon (20, 20a, 20b) has n wing portions (80, 80a, 80b), n being an integer of 2 or more, and n valley portions (90, 90a, 90b, 90c, 100, 100a, 100b, 100c), n being an integer of 2 or more, formed between the wing portions (80, 80a, 80b) adjacent thereto when the balloon (20, 20a, 20b) is folded around an outer periphery of the inner tube (50),
the n wing portions (80, 80a, 80b) and the n valley portions (90, 90a, 90b, 90c, 100, 100a, 100b, 100c) are arranged in a circumferential direction, and
a distance (X3, X5, X7) between at least one valley portion (100, 100a, 100b, 100c) of the n valley portions and the inner tube (50) is shorter than distances (X2, X4, X6) between other valley portions (90, 90a, 90b, 90c) and the inner tube (50).

2. The balloon catheter (10) according to claim 1, whereina portion where a distance (X5) between an n-th valley portion (100a), n being an integer of 3 or more, and the inner tube (50) is shorter than a distance (X4) between an (n-1)th valley portion (90a) adjacent to the n-th valley portion (100a) and the inner tube (50), and
a distance (X5) between an (n-2)th valley portion (100b) adjacent to the (n-1)th valley portion (90a) and the inner tube (50) is shorter than a distance (X4) between the (n-1)th valley portion (90a) and the inner tube (50).

3. The balloon catheter (10) according to claim 1 or claim 2, wherein
the n valley portions (90c, 100c) comprise a short valley portion (100c) having a short distance (X7) to the inner tube (50) and a long valley portion (90c) having a long distance (X6) to the inner tube (50), and
the short valley portion (100c) and the long valley portion (90c) are alternately arranged in a circumferential direction.

## Patentansprüche

1. Ballonkatheter (10) mit:
einem Innenrohr (50); und
einem am Innenrohr (50) befestigten Ballon (20, 20a, 20b), der zwischen einem gefalteten Zustand und einem ausgedehnten Zustand veränderbar ist,
**dadurch gekennzeichnet, dass**:
der Ballon (20, 20a, 20b), wenn er um den Außenumfang des Innenrohrs (50) herum gefaltet ist, n Flügel (80, 80a, 80b), wobei n eine ganze Zahl größer-gleich 2 ist, und n Hohlkehlen (90, 90a, 90b, 90c, 100, 100a, 100b, 100c), die zwischen nebeneinanderliegenden Flügeln (80, 80a, 80b) ausgebildet sind, wobei n eine ganze Zahl größer-gleich 2 ist, aufweist,
die n Flügel (80, 80a, 80b) und n Hohlkehlen (90, 90a, 90b, 90c, 100, 100a, 100b, 100c) in Umfangsrichtung angeordnet sind, und
ein Abstand (X3, X5, X7) zwischen wenigstens einer Hohlkehle (100, 100a, 100b, 100c) der n Hohlkehlen und dem Innenrohr (50) kleiner ist als Abstände (X2, X4, X6) zwischen den anderen Hohlkehlen (90, 90a, 90b, 90c) und dem Innenrohr (50).

2. Ballonkatheter (10) nach Anspruch 1, wobei
ein Abstand (X5) zwischen einer n-ten Hohlkehle (100a), wobei n eine ganze Zahl größer-gleich 3 ist, und dem Innenrohr (50) kleiner ist als ein Abstand (X4) zwischen einer (n-1)-ten Hohlkehle (90a), die neben der n-ten Hohlkehle (100a) liegt, und dem Innenrohr (50), und
ein Abstand (X5) zwischen einer (n-2)-ten Hohlkehle (100b), die neben der (n-1)-ten Hohlkehle (90a) liegt, und dem Innenrohr (50) kleiner ist als ein Abstand (X4) zwischen der (n-1)-ten Hohlkehle (90a) und dem Innenrohr (50).

3. Ballonkatheter (10) nach Anspruch 1 oder 2, wobei
die n Hohlkehlen (90c, 100c) eine tiefe Hohlkehle (100c) mit einem kleinen Abstand (X7) zum Innenrohr (50) und eine weniger tiefe Hohlkehle (90c) mit einem großen Abstand (X6) zum Innenrohr (50) aufweisen, und
die tiefe Hohlkehle (100c) und weniger tiefe Hohlkehle (90c) in Umfangsrichtung abwechselnd angeordnet sind.

## Revendications

1. Cathéter à ballonnet (10) comprenant :
un tube interne (50) ; et
un ballonnet (20, 20a, 20b) fixé au tube interne (50), le ballonnet (20, 20a, 20b) étant capable de transformer une transition entre un état plié et un état expansé,
**caractérisé en ce que** :
le ballonnet (20, 20a, 20b) a n parties formant ailes (80, 80a, 80b), n étant un entier de 2 ou plus, et n parties formant vallées (90, 90a, 90b, 90c, 100, 100a, 100b, 100c), n étant un entier de 2 ou plus, formées entre les parties formant ailes (80, 80a, 80b) adjacentes a celles-ci quand le ballonnet (20, 20a, 20b) est plié autour d'une périphérie externe du tube interne (50),
les n parties formant ailes (80, 80a, 80b) et les n parties formant vallées (90, 90a, 90b, 90c, 100, 100a, 100b, 100c) sont disposées dans une direction circonférentielle, et
une distance (X3, X5, X7) entre au moins une partie formant vallée (100, 100a, 100b, 100c) des n parties formant vallées et le tube interne (50) est plus courte que les distances (X2, X4, X6) entre d'autres parties formant vallées (90, 90a, 90b, 90c) et le tube interne (50).

2. Cathéter à ballonnet (10) selon la revendication 1, où une partie où une distance (X5) entre une n^{ième} partie formant vallée (100a), n étant un entier de 3 ou plus, et le tube interne (50) est plus courte qu'une distance (X4) entre une (n-1)^{ième} partie formant vallée (90a) adjacente à la n^{ième} partie formant vallée (100a) et le tube interne (50), et
une distance (X5) entre une (n-2)^{ième} partie formant vallée (100b) adjacente à la (n-1)^{ième} partie formant vallée (90a) et le tube interne (50) est plus courte qu'une distance (X4) entre la (n-1)^{ième} partie formant vallée (90a) et le tube interne (50).

3. Cathéter à ballonnet (10) selon la revendication 1 ou la revendication 2, où
les n parties formant vallées (90c, 100c) comprennent une courte partie formant vallée (100c) ayant une courte distance (X7) au tube interne (50) et une longue partie formant vallée (90c) ayant une longue distance (X6) au tube interne (50), et
la courte partie formant vallée (100c) et la longue partie formant vallée (90c) sont disposées de manière alternée dans une direction circonférentielle.
